# EUROPÄISCHE PATENTSCHRIFT

(11) **EP 0 775 442 B1**
(45) Veröffentlichungstag und Bekanntmachung des Hinweises auf die Patenterteilung: **24.10.2001**
(21) Anmeldenummer: 96114687.5
(22) Anmeldetag: 13.09.1996
(51) Int. Cl.: A01N 65/00, G01N 33/50

(54) **Pollen-Gemisch und seine Verwendung**
Pollen mixture and it's use
Mélange de pollen et son utilisation

(30) Priorität: 23.11.1995 DE 19543592
(43) Veröffentlichungstag der Anmeldung: 28.05.1997
(73) Patentinhaber: GSF-Forschungszentrum für Umwelt und Gesundheit GmbH, 85764 Oberschleissheim (DE)
(72) Erfinder: Bengsch, Eberhard, Prof. Dr., 81549 München (DE); Kettrup, Antonius, Prof. Dr., 59821 Arnsberg (DE); Polster, Jürgen, Prof. Dr., 85356 Freising (DE)

(56) Entgegenhaltungen:
- DE-A- 2 039 223
- GB-A- 1 082 159
- CHEMICAL ABSTRACTS, vol. 82, no. 21, 26.Mai 1975 Columbus, Ohio, US; abstract no. 135900, P. H. MOORE ET AL.: "Sugarcane pollen. I. In vitro germination of pollen." Seite 291; Spalte 2; XP002027163 & PHYTON (BUENOS AIRES), Bd. 32, Nr. 2, 1974, Seiten 147-157,
- CHEMICAL ABSTRACTS, vol. 77, no. 9, 28.August 1972 Columbus, Ohio, US; abstract no. 60760, E. N. KRASNOKUTSKAYA ET AL.: "Nutrient media for the germination of pollen from fruit-bearing plants." Seite 403; Spalte 2; XP002027164 & SB. NAUCH. TR., DONSKOI SEL'SKOKHOZ. INST., Bd. 6, Nr. 1(pt. 2), 1970, Seiten 87-89,
- CHEMICAL ABSTRACTS, vol. 74, no. 21, 24.Mai 1971 Columbus, Ohio, US; abstract no. 108263, K. TANABE ET AL.: "Physiological studies on germination of Japanese pear pollen (Pyrus serotina [Pyrus pyrifolia])" Seite 97; Spalte 2; XP002027165 & TOTTORI DAIGAKU NOGAKUBU KENKYU HOKOKU, Bd. 22, 1970, Seite 1-8

## Beschreibung

Die Erfindung betrifft ein Gemisch nach dem Oberbegriff des Patentanspruchs 1 und seine Verwendung.

Die Entwicklung von in vivo-nahen, tierversuchsfreien Testsystemen zur Bestimmung der biologische Toxizität von Substanzen ist eine wissenschaftliche und ethische Notwendigkeit allererster Wichtigkeit.

Das Auskeimen von Pollenkörnern und das anschließende Wachstum von Pollenschläuchen sind erwiesenermaßen borabhängig.

Die Borstimmulierung erlaubt es, den natürlicherweise auf dem borreichen Stigma stattfindenden Vorgang in vitro zu simulieren.

Bei den bisherigen Versuchen wurden Pollenkörner (z. B. Lilium longiflorum) in einer Lösung von klassischen Zuckern (Glucose, Saccharose) und Spuren von Borsäure aufgeschlämmt. Dabei kommt es partiell zu einer in vitro-Keimung unter Ausbildung mehr oder weniger langer Pollenschläuche. Dieser "Bor-Effekt" ist aus Th. Schmucker, Planta (Berlin) 18 (1933) 641-650 und Th. Schmucker, Planta (Berlin) 23 (1933) 264-284 bekannt.

Es mangelte seitdem nicht an Versuchen, dieses Phänomen zu einem Testsystem auf die Pollen-Vitalität und damit z. B. auf Qualität und Robustheit von Pflanzensorten zu entwickeln. Jedoch führte keiner dieser Ansätze zu einem praktisch realisierbaren Testsystem:

Unter den gegebenen Bedingungen erwies sich die in vitro-Pollenkeimung als hochstöranfällig, relativ langwierig und nicht reproduzierbar. Nur ein kleiner Teil der Pollen keimte aus, anschließend wuchsen die gebildeten Schläuche schleppend und erreichten nicht die Maximallänge. Da sich die Pollenkörner im Suspensionsmedium gegenseitig stimulieren, führten Starthemmungen eines Teiles von ihnen auch bei den unter diesen Bedingungen potentiell keimfähigen Körnern zu Störungen und damit schließlich zum Ausbleiben des Gesamteffekts. Das System war instabil und kaum quantifizierbar.

Andererseits bietet sich der Pollen als lebendes, rasch aktivierbares, hochansprechbares und natürlicherweise unbegrenzt verfügbares System für realitätsnahe Toxizitätsprüfungen geradezu an. Es sei daran erinnert, daß der Reproduktionsapparat und insbesondere der Pollen der empfindlichste Teil der Pflanze ist und am schnellsten und deutlichsten auf biologische, chemische und physikalische Toxizitäten reagiert und deshalb auch das beste Mittel darstellt, die biologische Wirkung solcher Toxizität quasi-systemidentisch zum lebenden Individium zu erfassen. Im Gegensatz zu Zellkultur-Linien, die im Verhältnis zum Gesamtorganismus ein hoch-amputiertes System darstellen, essentieller Interaktionen verlustig gegangen sind und nachgewiesenermaßen unter erhöhtem genomischen Streß stehen, ist der Pollen ein miniaturisiertes, komplettes, ruhendes und auf Wunsch aktivierbares Abbild des Gesamt-Individiums.

Die mühelose In-vitro-Steuerung eines solchen Systems stellt das ideale Arbeitsinstrument für realitäts-relevante Toxizitätsmessungen dar.

Aus Chemical Abstracts, Vol. 77, No. 9, 28. August 1972 Columbus, Ohio, US; Abstract No. 60760, E.N. Krasnokutskaya et al. und Chemical Abstracts, Vol. 74, No. 21, 24. Mai 1971 Columbus, Ohio, US; Abstract No. 108263, K. Tababe et.al. ist die Verwendung der Pollenaktivität bekannt. Die Verwendung von Zuckerkomponenten aus der L-Reihe ist dabei nicht erforderlich.

Aufgabe der Erfindung ist es, ein Gemisch der e. g. Art zur Verfügung zu stellen, mit dem Pollen zuverlässig und reproduzierbar stimuliert werden können.

Gelöst wird diese Aufgabe durch die Merkmale des Patentanspruchs 1. Die Unteransprüche beschreiben vorteilhafte Ausgestaltungen des Gemischs.

Die Ansprüche 5 bis 8 nennen vorteilhafte Verwendungen des Gemischs.

Es wird ein Verfahren beschrieben, welches erlaubt, die biologische Aktivität von Pollen in Ruhephase unter in vivo- sowie in vitro-Bedingungen in wirksamer Weise zu erwecken und kontrolliert zu amplifizieren. Bisher fanden für derartige Versuche Suspensionen von Pollen in wäßrigen Lösungen von anorganischen oder organischen Borverbindungen und Sacharose oder Glukose Verwendung. Dieses System blieb ohne praktische Einsatzfähigkeit als Test. Die erfindungsgemäße entscheidende Verbesserung besteht darin, dem Gemisch als glykosidische Komponente anstelle der klassischen D-Zucker solche der L-Serie und außerdem Aluminiumverbindungen zuzufügen:

Das vormals nur ansatzmäßig als Test verwendbare System erlangt auf diese Weise einen hohen Grad an Empfindlichkeit, Regulierbarkeit, Reproduzierbarkeit und Universalität. Dies erlaubt nunmehr seine technische Anwendung
- als Schnell-Testsystem auf biologische Toxizität von Substraten aller Art
- als Indikator des integralen Schädigungsgrades der vegetalen Umwelt anhand der Aktivität von aus dem Untersuchungssektor stammenden Pollen
- zur Verbesserung der Befruchtungseffizienz zwecks Erlangung von mehr und besseren Produkten in der Agri- und Horti-Kultur sowie in der Gentechnologie.

Der rasche, mit geringstem Aufwand funktionierende Test betrifft Chemie, Biochemie, Umwelt und Fertilitätsprobleme in der Landwirtschaft.

Erfindungsgemäß wurde festgestellt, daß die gewünschte Wirkung mit Hilfe von 3 Wirksubstraten erzielt werden kann:
1) Zucker der L-Reihe, beispielsweise L-Fucose, sorgen für ein rasches, sicheres und reproduzierbares Wachstum der Pollenschläuche.
2) Aluminiumionen sorgen für eine hohe Auskeimungsrate der Pollenkörner und stimulieren angeschwächte Pollen zu regulärer kontrollierter Aktivität. Sie nivellieren Unterschiede in den Individuen.
3) Bor bewirkt die prinzipielle Keimfähigkeit von Pollenkörnern außerhalb des in -vio -Systems

Durch die erfindungsgemäße Substratmischung wird es möglich, Pollen-Aktivitätsmessungen unter definierten Standard-Bedingungen auszuführen und auf alle gewünschten Probleme anzuwenden.

Die Schlüsselrolle der Substrate Aluminium und L-Zucker für die prä-fekondative Pollen-Aktivierung ist umso überraschender, als
- einerseits Aluminium in der Pflanzenphysiologie bisher ausschließlich als toxischer Parameter betrachtet wurde (z. B. Diskussion zum Waldsterben durch Aluminiumzufuhr aus übersäuerten Böden)
- andererseits in der Glykosidchemie der vegetalen Befruchtungsvorgänge die gesamte Stoffklasse der natürlicherweise unüblichen L-Zucker bisher von den Betrachtungen ausgeschlossen blieb.

Zum Verständnis des Phänomens und seiner optimalen Anwendung war es erst einmal notwendig, verschiedene Teile des pflanzlichen Reproduktionsapparates auf ihren Eigengehalt an den beschriebenen Schlüsselsubstanzen und ggf. anderen Wirksubstraten zu untersuchen. Unter Zuhilfenahme sporadisch vorhandener Literaturangaben ergab sich dabei folgendes Bild:

Auffallend hohe Werte für die an dem Testsystem beteiligten Wirksubstrate wurden selektiv in den generativen Teilen der Blüten (Pollen, Stigma, Fruchtknoten) gefunden, wo sie offensichtlich eine wichtige Rolle spielen. Dies bestätigt die erfindungsgemäß unter in vitro-Bedingungen entdeckten Effekte.

So enthalten beispielsweise Pollen von Prunus avia und Tulipa von sich aus 80 bzw. 50 ppm Aluminium, was im Verhältnis zur Biomasse der Gesamtpflanze eine hohe selektive Anreicherung bedeutet.

Die gleichen Kumulationseffekte werden für das prä-erfindungsmäßig zum Testsystem gehörige Bor beobachtet. In der Blüte der von uns vorzugsweise verwendeten Art Lilium longiflorum befanden sich natürlicherweise folgende Mengen an Bor (bezogen auf die Trockenmasse):
Pollen 100 ppm
Stigma 60 ppm
Fruchtknoten 15 ppm.

In durch verstärkte Borzufuhr angereicherten Pflanzen stiegen diese Werte selektiv um das 20- bis 50-fache an.

Die Erfindung wird im folgenden anhand von Ausführungsbeispielen näher erläutert.

Beim ersten Ausführungsbeispiel für einen in vitro-Toxizitätsschnelltest wird beispielsweise wie folgt verfahren:

### Kontrolltest

Ungefähr 500 frisch von der Anthere von Lilium l abgestreifte Pollenkörner werden in 1 ml einer 2%igen Lösung von L-Fucose aufgeschlämmt. Die Lösung enthält weiterhin ca. 5 ppm Aluminium (AlCl₃) und ca. 5 ppm Bor (Borsäure). Die Al Konzentration kann zwischen 0,1 und 20 ppm liegen, der optimale Bereich liegt zwischen 3 und 7 ppm und die Borkonzentration kann zwischen 1 und 20 ppm gewählt werden, optimal ist ein Bereich zwischen 3 und 7 ppm. Der pH-Wert des Testsystems liegt bei 6. Die Temperatur zwischen 18 und 25° C. Nach ca. 1 Stunde ist bei diesem Kontrollsystem bereits die Hälfte der Pollenkörner angekeimt. Die Pollenschläuche wachsen rasch aus und erreichen Längen zwischen 1 - 10 mm. Dies wird in einer graduierten Meßzelle mikroskopisch quantitativ ausgewertet. Zur Quantifizierung können sowohl die Dynamik als auch der nach 5-10 Stunden erreichte Endzustand oder beide ausgemessen werden.

Durch die Gegenwart von Aluminium gewinnt das System einen hohen Grad an Konstanz und Reproduzierbarkeit: Partielle Schädigungen der empfindlichen Pollen haben keinen Einfluß auf die Auskeimungszahlen, außerdem ist diese ca. 10 x so hoch wie bei der vorerfindungsgemäßen Abwesenheit von Aluminium.

Die Verwendung von Zuckern der L-Serie, vorzugsweise L-Fucose, anstelle der üblichen D-Zucker bewirkt
- eine starke Beschleunigung des Schlauchwachstums
- eine hohe Funktionalität auch bei partiell geschädigten Körnern.

Sinvolle Konzentrationen an L-Zuckern im Gemisch sind zwischen 0.5 und 5%.

Zur raschen und effizienten Auswertung wurden die Pollenschläuche vier Längenbereichen zugeordnet (kurz, mittel, lang, sehr lang). Auf diese Weise wurde der Effekt in eine Zahl für die mittlere Pollenschlauchlänge konvertiert. Auskeimungszahl x mittlere Pollenschlauchlänge ergeben so den gewünschten quantitativen Meßparameter für die Pollenaktivität (Quantitative Mikroskopie).

Insgesamt bleiben die Fluktuationen in dem erfindungsgemäßen Testsystem gering, was eine hohe Reproduzierbarkeit garantiert.

Hingegen war die vorerfindungsgemäße Anordnung (Verwendung üblicher D-Zucker in Abwesenheit von Aluminium) bereits im Kontrollgang von so hohen individuellen Unsicherheiten behaftet, daß jeder gesuchte Toxizitätseffekt von statistischen Schwankungen überdeckt wurde.

### Toxizitäts-Schnelltest

Es wurden beispielsweise Substanzen aus zwei öko-toxischen Problemgruppen herangezogen:
- Schwermetalle (Zink, Blei und Platinverbindungen)
- Pestizide (Dicuran)

Für die Bestimmung der biologischen Toxizität von Zinkverbindungen wurden dem Test-Set zusammen mit den Standard-Pollen 2 ppm Zink (ZnSO₄) zugefügt. Aus der beschriebenen quantitativen Mikroskopie ergab sich eine Aktivitätsreduzierung um den Faktor 3, was für solche Konzentrationen, die im Trinkwasser noch zugelassen sind, bereits eine akute Toxizität indiziert.

Auch für Platinsalze (100 ppb) wurde eine signifikante Toxizität nachgewiesen. Bei der allgemeinen Verwendung des Platins als Abgaskatalysator weist dieser Befund frühzeitig auf ein augenblicklich neu entstehendes ökologisches Problem hin.

Auch sonst reagieren Pollen erwiesenermaßen sehr empfindlich auf Umweltchemikalien aller Art. Die Gegenwart von Harnstoff-Herbiziden, wie beispielsweise Dicuran (20 ppm), bewirkte im Testsystem eine Totalhemmung.

Die Toxizität von Schadstoffmengen, die bisher als unterschwellig betrachtet wurden, wird auf diese Weise meßbar dokumentiert.

Das erfindungsgemäße Schnell-Testverfahren kann in vielfältiger Weise abgewandelt und an spezielle Probleme angepaßt werden:
- Grundsätzlich sind alle Pollenarten anwendbar. Versuche wurden mit Lilium und Antirrhinum majus, Tulipa und einer Reihe von bei der Insekten-Bestäubung wichtigen und besonders robusten Pollenarten unternommen (Genista, Crataegus).
   Als weitere zusätzliche Pollenaktivierungssubstrate wurden die Effekte von Siliziumverbindungen (neutralisiertes Wasserglas) und einigen natürlicherweise in den Blüten vorhandenen Polyphenolen wie Quercitin, Vitexin, Chlorogensäure, Rosmarinsäure und Kaffeesäure vermessen.
   Sie kommen als Zusatzkomponenten für auf spezielle Probleme erarbeitete Varianten des Testsystems in Frage.
   Eine solche spezielle Ausführungsform des Testsystems ist beispielsweise die rasche, hochempfindliche und frühzeitige Quantifizierung des Schädigungsgrade von Pflanzen durch UV - B -Licht. Lange bevor die Schadensphänomene an der Gesamtpflanze erscheinen ( Blattschwellung, Formänderung, Nekrotisierung ), ist die Pollenaktivität bereits signifikant verändert. Der definierte Zusatz von Polyphenolen bzw. photodynamisierenden Molekülen wie Hypericin erlaubt es, die Sensibilität des Standard-Testsystems in der einen oder in der anderen Richtung zu verschieben.
- Als Glykosidkomponente finden vorteilhaft auch andere L-Zucker wie Rhamnose, Arabinose, Galaktose, Mannose oder Mischungen daraus Verwendung.
   Allgemein erlauben es das Schnell-Testverfahren und seine Varianten, Umweltproben, Chemikalien und biochemische Produkte aller Art schnell und preiswert auf ihre biologische Toxizität zu testen. Da der Reproduktionsapparat von Pflanzen am empfindlichsten auf Umwelteinflüsse reagiert, sind Schnelltests auf Pollenbasis zur Abschätzung der biologischen Toxizität von Umweltchemikalien prädestiniert. Um aber nicht nur unsichere Andeutungen und/oder einfache ja/nein-Entscheidungen (ja: toxisch, nein: nicht-toxisch) zu erhalten, sondern auch quantitative Beurteilungen, sind die erfindungsgemäß optimierten Nährmedien für das Pollenschlauchwachstum unerläßlich. Nur so kann die volle Breite der Toxizitätsskala erfaßt werden.
   Das beschriebene Testverfahren ist rasch, leicht ausführbar und ungewöhnlich preiswert, und dies insbesondere im Vergleich zu Toxizitätsprüfungen z. B. an wachsenden Pflanzen, die zusätzlich noch saisonabhängig sind. Pollenkörner hingegen können eingefroren und zu jeder Zeit reaktiviert werden.

Da Pollen repräsentativ für die integrale Umweltschädigung sind, kann das Testverfahren mühelos zur Indikation und zur Quantifizierung dieses Zustandes verwendet werden. Zu diesem Zweck werden umgekehrt nicht die auf Toxizität zu prüfenden Testlösungen variiert, sondern die zu untersuchenden Pollenproben. Die Aktivität gesunder Standard-Pollen wird mit der von Pollen aus dem Untersuchungssektor in dem beschriebenen Standard-Testsystem vermessen und verglichen.

Durch die ubiquitäre Applikation von Pestiziden in Landwirtschaft, Gartenbau sowie in der Forstwirtschaft wird eine Abnahme der Pollenvitalität und Aktivität immer häufiger beobachtet. Daher ist es zunehmend bedeutungsvoller, die Bestäubungs- und Befruchtungsbedingungen biochemisch zu optimieren.

Die Kenntnis der Stoffe, die unter natürlichen Bedingungen zentral den Bestäubungs- und Befruchtungsvorgang bei Pflanzen steuern, ist von großer wirtschaftlicher Bedeutung. Dadurch können Ernteausfälle vermieden und Züchtungserfolge verstärkt werden. Die erfindungsgemäße Testlösung kann zwecks einer solchen Erhöhung der Züchtungserfolge als Sprühmittel auf blühendes Pflanzenmaterial gebracht werden, was besonders bei teuren Pflanzensorten und bei genetisch veränderten Pflanzen, wo oft nur wenige Individuen eines gewünschten Phänotyps zur Verfügung stehen, die anschließend unter optimierten Bedingungen vermehrt oder gekreuzt werden müssen.

## Patentansprüche

1. Gemisch, bestehend aus einer Suspension von Pollen in einer Lösung, die als Mindestbestandteile Zucker und Borverbindungen enthält, **dadurch gekennzeichnet, daß** die Zuckerkompo-nente aus Zuckern der L-Reihe gebildet wird und daß Aluminiumverbindungen als Aktivierungssubstrat hinzugefügt werden.

2. Gemisch nach Anspruch 1, **dadurch gekennzeichnet, daß** die Zucker vorzugsweise L-Fucose, L-Rhamnose, L-Arabinose, L-Galaktose oder jeder andere Zucker der L-Reihe sowie ein Gemisch aus diesen sind.

3. Gemisch nach Anspruch 1 oder 2, **dadurch gekennzeichnet, daß** die Konzentration an L-Zuckern 0,5 - 5 % beträgt.

4. Gemisch nach einem der Ansprüche 1 bis 3, **dadurch gekennzeichnet, daß** die Konzentration an Aluminium 0,1 - 20 ppm beträgt.

5. Verwendung des Gemischs gemäß einem der Ansprüche 1 bis 4 zur Steigerung der Pollenaktivität.

6. Verwendung des Gemischs gemäß einem der Ansprüche 1 bis 4 zur Bestimmung der biologischen Toxizität von Chemikalien.

7. Verwendung des Gemischs gemäß einem der Ansprüche 1 bis 4 zur Bestimmung und und Quantifizierung der integralen Umweltschädigung in gegebenen geographischen Sektoren.

8. Verwendung des Gemischs gemäß einem der Ansprüche 1 bis 4 als Sprühmittel zur Optimierung bei Bestäubungs- und Befruchtungsvorgängen in Pflanzen, oder bei gen-technisch veränderten Pflanzen.

## Claims

1. Mixture, comprising a suspension of pollen in a solution which contains at least sugar and boron compounds, **characterised in that** the sugar component is formed from sugars of the L series, and **in that** aluminium compounds are added as an activating substrate.

2. Mixture according to claim 1, **characterised in that** the sugars are preferably L-fucose, L-rhamnose, L-arabinose, L-galactose or any other sugar of the L series, as well as a mixture thereof.

3. Mixture according to claim 1 or 2, **characterised in that** the concentration of L sugars is 0.5 - 5 %.

4. Mixture according to any one of claims 1 to 3, **characterised in that** the concentration of aluminium is 0.1 - 20 ppm.

5. Use of the mixture according to any one of claims 1 to 4 for increasing the pollen activity.

6. Use of the mixture according to any one of claims 1 to 4 for determining the biological toxicity of chemicals.

7. Use of the mixture according to any one of claims 1 to 4 for determining and quantifying the integral damage to the environment in given geographical sectors.

8. Use of the mixture according to any one of claims 1 to 4 as a spraying agent for optimising pollination and fertilisation processes in plants or genetically modified plants.

## Revendications

1. Mélange constitué d'une suspension de pollen dans une solution qui contient comme constituants minimum des sucres et des composés de bore,
**caractérisé en ce que**
le composant sucre est formé de sucres de la série L et des composés d'aluminium sont ajoutés comme substrats d'activation.

2. Mélange selon la revendication 1,
**caractérisé en ce que**
les sucres sont de préférence le L-fucose, le L-ramnose, le L-arabinose, le L-galactose ou n'importe quel autre sucre de la série L ainsi qu'un de leurs mélanges.

3. Mélange selon la revendication 1 ou 2,
**caractérisé en ce que**
la concentration en sucres de la série L va de 0,5 à 5 %.

4. Mélange selon l'une des revendications 1 à 3,
**caractérisé en ce que**
la concentration en aluminium va de 0,1 à 20 ppm.

5. Utilisation des mélanges selon l'une des revendications 1 à 4, pour l'accroissement de l'activité des pollens.

6. Utilisation du mélange selon l'une des revendications 1 à 4, pour la détermination de la toxicité biologique des produits chimiques.

7. Utilisation du mélange selon l'une des revendications 1 à 4, pour la détermination et la quantification de l'atteinte intégrale de l'environnement dans des secteurs géographiques donnés.

8. Utilisation du mélange selon l'une des revendications 1 à 4 comme produit de pulvérisation aux fins d'optimisation dans le processus de pollinisation et de fructification dans les plantes, ou dans des plantes modifiées par génie génétique.
